# EUROPÄISCHE PATENTSCHRIFT

(11) **EP 0 298 121 B1**
(45) Veröffentlichungstag und Bekanntmachung des Hinweises auf die Patenterteilung: **21.04.1993**
(21) Anmeldenummer: 87902189.7
(22) Anmeldetag: 14.01.1987
(51) Int. Cl.: A61L 15/00, A61L 15/16

(54) **BIOVERTRÄGLICHES MATERIAL ZUR BEHANDLUNG VON FEHLERN IN GEWEBEN UND ORGANEN**
BIOCOMPATIBLE MATERIAL FOR TREATMENT OF DEFECTS IN TISSUES AND ORGANS
MATERIAU BIOCOMPATIBLE POUR LE TRAITEMENT DE DEFAUTS DANS LES TISSUS ET LES ORGANES

(43) Veröffentlichungstag der Anmeldung: 11.01.1989
(73) Patentinhaber: VSESOJUZNY NAUCHNO-ISSLEDOVATELSKY I ISPYTATELNY INSTITUT MEDITSINSKOI TEKHNIKI, Moscow, 129301 (RU); MOSKOVSKY NAUCHNO-ISSLEDOVATELSKY ONKOLOGICHESKY INSTITUT IMENI P.A. GERTSENA, Moscow, 125284 (RU)
(72) Erfinder: AKIMOVA, Alla Yakovlevna, Moscow, 125502 (SU); DAVYDOV, Anatoly Borisovich, Moscow, 111395 (SU); EGIEV, Valery Nikolaevich, Moscow, 109125 (SU); ILIINA, Anna Ivanovna, Pushkino, 141200 (SU); KAPUSTIN, Jury Pavlovich, Moscow, 101000 (SU); ORFANIDI, Anesty Khristovich, Moscow, 125502 (SU); SMIRNOV, Alexei Nikolaevich, Moscow, 123308 (SU); STEPANOV, Eduard Alexandrovich, Moscow, 117321 (SU); TIMOKHINA, Valeria Iosifovna, Moscow, 129348 (SU); CHIGIR, Anatoly Nikolaevich, Moscow, 119501 (SU); SCHITKOV, Kirill Georgievich, Moscow, 105523 (SU)
(74) Vertreter: von Füner, Alexander, Dr.
(86) Internationale Anmeldenummer: SU8700002
(87) Internationale Veröffentlichungsnummer: WO8805311

(56) Entgegenhaltungen:
- GB-A- 1 221 282
- US-A- 3 630 194
- US-A- 4 316 457

## Beschreibung

Die vorliegende Erfindung bezieht sich auf das Gebiet der Medizin, insbesondere auf ein biokompatibles Material zur Behandlung von Gewebe- und Organdefekten.

Bekannt ist eine Mischung für Bindelemente der Weichteile von Geweben und Organen auf der Grundlage eines resorptionsfähigen Polymeres, die ein Copolymeres von N-Vinylpyrrolidon und Alkylestern von Acryl- und/oder Methacrylsäuren enthält, welche im Alkylrest 2 bis 8 Kohlenstoffatome enthalten (BE-PS 899078). Die Bindeelemente stellen Platten und Folien mit vorgegebener Konfiguration dar und werden vorwiegend bei der Koinzidenz und hermetischen Abdichtung geschnittener Teile von parenchymatösen Organen verwendet. Die Resorption der Folie im Organismus erfolgt in 35 bis 250 Tagen, die Bruchfestigkeit beträgt von 6,7 bis 18,8 kp/cm², die bezogene Dehnung nach 30minütigem Halten in einer physiologischen Lösung (welche die Elastizität im Anschwellungszustand charakterisiert), beträgt von 21 bis 880 %.

Jedoch kennzeichnen sich die genannten Mischungen dadurch aus, daß sie im Trockenzustand in Form von Folien brüchig sind, und es ist notwendig, die Folien vor der Anwendung 10 bis 15 Minuten in einer physiologischen Lösung zu halten, damit sie elastisch werden.

Die Erhöhung der Elastizität der Folien (die sich durch bezogene Dehnung kennzeichnet) bewirkt eine Senkung der Festigkeit des Materials. Die Folieresorption dauert länger als die Regeneration der Weichteile. Daher wachsen die Gewebe nicht in die Bindeelemente hinein.

Darüber hinaus ermöglicht dieses Material für Bindeelemente es nicht, die Absorptionsfähigkeit, die sich durch den Anschwellungsgrad kennzeichnet, zu regeln. Daher kann das Material nicht zur Behandlung von eitrigen Haut- und Operationswunden verwendet werden.

Bekannt ist ferner das Material "Debrisan" (Firma "Pharmacea", Schweden), das zur Behandlung von eitrigen Wunden ("Khirurgia", Verlag Meditsina, Moskau Nr. 8, 1982, S. 88 -91) verwendet wird. Debrisan stellt ein weißes Pulver dar, das aus Dextranpolymeren besteht. Debrisan besitzt eine hohe Absorptionsfähigkeit und zwar nimmt 1 g Präparat bis zu 4 ml Flüssigkeit auf. Debrisan schüttet man in die Wunde und legt, einen feuchten Tupfer, ein Umschlagpapier darauf. Die Umschläge werden täglich gewechselt; dabei wird das Pulver mit Gazekugeln entfernt und mit irgendeiner Lösung abgespült. Die Anwendung von Debrisan trägt zur Wundreinigung und -eintrocknung, und zur Senkung der Ödematose bei. Sind in der Wunde Pyozyaneusbakterien vorhanden oder ist diese sehr vereitert, erweist sich die Anwendung von Debrisan als uneffektiv. In einem solchen Fall ist die Wirkung von antibakteriellen Präparaten in den ersten 2 bis 3 Tagen effektiver.

Bei der Versorgung tiefer Wunden und von Fistelgängen treten auch Schwierigkeiten beim Auftragen und bei der Entfernung von Debrisan auf.

Der Erfindung liegt die Aufgabe zugrunde, durch Änderung seiner Zusammensetzung ein biokompatibles Material zu erhalten, das eine regulierbare Resorptionsfähigkeit, eine regulierbare Resorptionsgeschwindigkeit, welche mit der Geschwindigkeit der Geweberegeneration übereinstimmt, sowie eine erhöhte Elastizität bei Aufrechterhaltung einer hohen Festigkeit aufweist.

Die Aufgabe wurde dadurch gelöst, daß sich ein biokompatibles Material zur Behandlung von Gewebe- und Organdefekten auf der Grundlage eines resorptionsfähigen Polymeren erfindungsgemäß aus Pfropfcopolymerem von α-Cyanacrylat mit Polyacrylsäure mit einer Molekularmasse von 200 000 bis 600 000 besteht, das durch Umsetzung von α-Cyanacrylat mit Polyacrylsäure in Anwesenheit eines Vernetzungsmittels bei folgendem Verhälnis der genannten Komponenten in Masse% erhalten worden ist:

| | |
|---|---|
| α-Cyanacrylat | 14 - 34 |
| Polyacrylsäure mit einer Molekularmasse von 200 000 bis 600 000 | 15 - 43 |
| Vernetzungsmittel | 71 - 23 |

Das erfindungsgemäße biokompatible Material enthält als α-Cyanacrylat vorzugsweise Ethyl-α-Cyanacrylat, Ethoxethyl-α-Cyanacrylat, Butyl-α-Cyanacrylat oder deren Gemische.

Als Vernetzungsmittel enthält das erfindungsgemäße biokompatible Material eine makromolekulare Raummatrix aus Polyvinylalkoholblöcken mit einer Molekularmasse von 50.000 bis 60.000, Glyceringliedern oder Gemische dieser Glieder. Das erfindungsgemäße biokompatible Material kann in Form von Granulat mit einem Durchmesser von 0,5 bis 1,5 mm, in Form von Fäden mit einem Durchmesser von 0,8 - 2,5 mm, in Form von Folien mit einer Dicke von 100 bis 1000 µm, in Form von Platten mit einer Dicke von 10 bis 50 mm, von Platten mit einer entwickelten Oberfläche erhalten werden, die die maximal große Fläche für den Kontakt mit einer Wunde ermöglicht.

Die Materialdichte (ρ) beträgt 0,16 - 0,8 g/cm³, die Absorptionsfähigkeit, die sich durch eine Masseanschwellung (A) in 24 Stunden charakterisiert, 500 - 15 000 %, die bezogene Dehnung (ε), die die Elastizität charakterisiert, 10 - 15 %, und die Bruchfestigkeit 40-55 kp/cm². Unter der Einwirkung von Medien des Organismus vollziehen sich eine Fragmentation und biologische Degradation des Materials.

Der Anschwellungsgrad des biokompatiblen Materials kann 10 000 - 15 000 % erreichen, wodurch es aussichtsreich für die Behandlung von eitrigen Haut- und Operationswunden wird.

Wenn notwendig, können Arzneimittel, z.B. Antiseptika in das biokompatible Material eingeführt werden. Aus diesem Grunde kann es in der Chirurgie und Traumatologie zur Verkürzung und Vorbeugung der Infizierung angewandt werden. Vorzugsweise enthält das erfindungsgemäße biokompatible Material ein Antiseptikum in einer Menge von 9 bis 50 Masse%.

Das erfindungsgemäße biokompatible Material zeichnet sich durch eine Resorptionsdauer, welche seine ständige Substituierung durch regenerierendes Gewebe des Organismus gewährleistet aus. Im Rahmen der Grenzwerte der genannten Rezeptur entsteht ein biokompatibles Material, das sich durch eine ziemlich hohe Festigkeit und Elastizität sowohl im Trocken- als auch in angeschwollenem Zustand auszeichnet, wodurch es sich auf Oberflächen mit Formprofil leicht formen läßt. Der Anschwellungsgrad des biokompatiblen Materials, der seine Absorptionsfähigkeit kennzeichnet, kann 15 000 % im Laufe von 24 Stunden erreichen, wodurch das Material für die Behandlung von eitrigen Haut- und Operationswunden geeignet wird.

Das biokompatible Material, das nach Rezepturen erhalten wird, welche die genannten Grenzwerte überschreiten, löst nach seinen Charakteristiken nicht die gestellte Aufgabe. Ein Material, das durch Umsetzung wäßriger Lösungen von Polyacrylsäure und Polyvinylalkohol mit α-Cyanacrylat, genommen in einer Menge von weniger als 14 %, erhalten ist, erleidet bereits in 6 Stunden eine Fragmentation und Biodegradation und kann nicht zur Schließung und Behandlung von Defekten der Gewebe- und Organweichtelle verwendet werden. Ein biokompatibles Material, das unter Anwendung von α-Cyanacrylaten in einer Menge von über 34 % erhalten wurde resorbiert im Organismus im Laufe von über 2 Monaten und behindert daher seine ununterbrochene Substitution mit regenerierendem Gewebe und kann nicht zur Schließung und Behandlung von Defekten der Gewebe- und Organweichteile verwendet werden.

Das erfindungsgemäße biokompatible Material wurde bei einem Experiment an männlichen Ratten "Vistar" mit einem Gewicht von 200 - 240 g untersucht. Die Experimente wurden unter Inhaltions-Ethernarkose durchgeführt. Nach einem 1,2-2,0 cm langen Hauteinschnitt auf der Vorderbauchwand nach obtuser Methode schied sich die Haut von unterliegenden Muskeln ab; in die entstandene Tasche führte man eine 1,0 - 1,5 cm lange Folie ein. Die Ergebnisse des Experiments zeigten daß am 7. Tage nach der Implantation eine starke Wucherung des Granulationsgewebes um die Folie herum sowie zwischen den Muskelfasern zu beobachten ist. An der Seite des Implantats wird das Gewebe kollagen. Um die Folie herum zeichnet sich die Bildung einer bindgewebigen Kapsel ab, die sich am 21. Tage nach der Operation endgültig herausbildet und mit den darunterliegenden Muskeln sowie stellenweise mit der Haut eng verwachsen ist.

Die Biodeskruktion der Folie, die gleichzeitig mit dem Beginn der Herausbildung einer bindegewebigen Kapsel anfängt, endet praktisch am 21. - 23. Tage nach der Operation.

Histologisch wird in den Gebieten des Anliegens der Kapsel an den Muskel das Hineinwachsen ihrer Elemente zwischen den einzelnen Muskelfasern nachgewiesen. Die Implantation des Materials wird nicht von der Entwicklung schroffer dystrophischer oder nekrobiotischer Modifikationen im darunterliegenden Muskelgewebe begleitet.

Das erfindungsgemäße biokompatible Material wurde für die Abdeckung der Bauchspeicheldrüse im Versuch an 10 geschlechtsreifen Ratten mit einem Gewicht von 200 bis 250 g erprobt. Das Experiment wurde unter der Inhalations-Ethernarkose durchgeführt. Es wurde eine supramediane Laparotomie vorgenommen. Durch die Wunde führte man den Zwölffingerdarm, den Magen, die Bauchspeicheldrüse und die Milz heraus. Auf die Bauchspeicheldrüse legte man eine Folie mit einer Fläche von 2 x 3 cm². Danach wurde die Drüse in die Bauchhöhle eingetaucht, und die Operationswunde wurde schichtweise vernäht. Die Ratten wurden zu verschiedener Zeit nach der Operation für makroskopische und histologische Untersuchungen aus dem Experiment genommen. Drei Wochen nach der Operation wurde das Fehlen der Folie in der Bauchhöhle festgestellt. Die makroskopische Bauchspeicheldrüse war nicht verändert; Adhäsionen waren in der Bauchhöhle nicht zu beobachten. Bei einer histologischen Untersuchung wurde eine normale Struktur des Organs bestimmt; Entzündungsveränderungen fehlten.

Erforscht wurde auch die Möglichkeit der Verwendung des erfindungsgemäßen biokompatiblen Materials mit einer Armierung in Form eines Lavsannetzes zur Substitution des eingebüßten Schließapparats des Dickdarms bei Kindern mit neurogenen Störungen von Beckenorganen. Ein Experiment nahm man an 10 Katzen nach folgender Methodik vor. Abgesondert wurde der distale Abschnitt des Mastdarms, der mit einem Streifen des biokompatiblen Materials umgeben wurde, welcher an die Schambeine angenäht wird. Dadurch wurde der Mastdarm nach vorn gezogen, wodurch ein Schließapparat entstand. Eine morphologische Untersuchung im Laufe von 1 bis 3 Monaten nach der Operation zeigte, daß das biokompatible Material durch ein Narben-Bindegewebe ersetzt wird, das später den Mastdarm auch schließt. In einer Klinik wurde eine ähnliche Operation an 8 Kranken mit völliger Stuhlinkontinenz durchgeführt. Das Ergebnis war in allen Fällen positiv.

Bei Erprobungen des erfindungsgemäßen biokompatiblen Materials, das eine hohe Resorptionsfähigkeit (die Anschwellung im Laufe von 24 Stunden 5 000 - 15 000 %) auf flüssigen Nährböden besitzt, wurde eine gleichmäßige Absorptionsfähigkeit von Mikroorganismen entdeckt, die sich über die gesamte Dicke des Materials erstreckt. Die erhaltenen Angaben sind eine Voraussetzung für die klinische Anwendung des biokompatiblen Materials bei Kranken zur Dränage eitriger,flacher Hautwunden.

Zu diesem Zwecke wurde das erfindungsgemäße biokompatible Material verwendet, das Antiseptika, z.B. Chlorophyllit (Eukalyptusblätterextrakt, enthaltend eine Chlorophyllmischung) enthält. Klinisch wandte man das biokompatible Material, das ein Antispetikum enthält, bei 15 Kranken an. In allen Fällen erhielt man einen ausgeprägten klinischen Effekt - eine schnelle Reinigung der Wunde von eitrigem Belag, eine Änderung der Mikrobenzahl, ein schnelles Aufkommen von Granulationen und Randepithelisation und eine Kürzung der Dauer der Wundheilung im Vergleich mit traditionellen Behandlungsmethoden.

Der plastische Ersatz von extensiven Defekten der vorderen Bauchwand unter Anwendung des erfindungsgemäßen biokompatiblen Materials in Form von Platten mit armierendem Lavsannetz wurde bei einem Experiment an 50 Ratten im Alter von 2-2,5 Monaten, mit einem Gewicht von 150 g untersucht. Durch Ausschnitte rief man vier Arten von Defekten der vorderen Bauchwand in Durchlaß- und Querrichtung hervor, die 20 und 40 % der Fläche einnehmen. Danach wurde die Defektstelle mit einem ovalen Teil des biokompatiblen armierten Materials bedeckt, wobei die Haut darüber eingenäht wurde. Die Ergebnisse haben ergeben, daß die Heilung der Operationswunde auf der Haut per primam intentionem erfolgte. Bei einer morphologischen Untersuchung der entfernten Präparate der vorderen Bauchwand der Ratten im Laufe von 1,2 und 3 Monaten nach der Operation wurde einer guter Ersatz des erfindungsgemäßen biokompatiblen Materials durch Bindegewebe ohne Anzeichen eines Entzündungsprozesses nachgewiesen. Von der Seite der Bauchhöhle her fehlten Erscheinungen einer Verwachsung von Darmschlingen mit der vorderen Bauchwand; die innere Fläche des substituierten Defekts hatte eine dem Bauchfell ähnliche Auskleidung mit entwickeltem Netz von kleinen Gefäßen. In einer Klinik wurde eine ähnliche Operation an 6 Kranken mit Embryonalhernien und Gastroschisis durchgeführt. In allen Fällen gelang es, die Defektstelle der vorderen Bauchwand völlig zu schließen. Die Operationszeit konnte wesentlichen gekürzt werden.

Das biokompatible Material, bestehend aus einem Propfcopolymeren von α-Cyanacrylat mit Polyacrylsäure, erhält man durch Vermischen einer Lösung von α-Cyanacrylat in einem organischen Lösungsmittel, das keine Polymerisation von α-Cyanacrylat hervorruft und mit Wasser inkompatibel ist, mit einer wäßrigen Lösung von Polyacrylsäure mit einer Molekularmasse von 200 000 bis 600 000 und eines Vernetzungsmittels, vorzugsweise Polyvinilalkohols mit einer Molekularmasse von 50 000 bis 60 000, oder Glycerins oder deren Mischung bis zur Bildung einer Emulsion. Das erfindungsgemäße biokompatible Material in Form von Folien und Platten erhält man aus der Emulsion nach der Begießungsmethode, in Form von Granulat durch Tröpfeln der Emulsion in ein Fällbad (Elektrolytlösung), und in Form von Fäden - durch Auspressen durch ein Profilgießtrichter in ein Fällbad. Die Lösungsmittel werden in an sich bekannter Weise entfernt, beispielsweise durch Halten des Erzeugnisses in einem bestimmten Temperaturenbereich. Nach dem Trocknen kann das Erzeugnis aus dem biokompatiblen Material in Form von Folien, Platten, Granulat und Fäden zusätzlich einer Wärmebehandlung ausgesetzt werden, damit sich vernetzte Polymere bilden.

Die Kurven einer Differentialthermoanalyse von Folien, die nach der genannten Methode erhalten werden, bestätigen eine Umsetzung zwischen α-Cyanacrylaten und Polyacrylsäure und unterscheiden sich von den Kurven für Homopolymere (Polyethyl-α-cyanacrylat, Polyacrylsäure und ihre heterogenen Gemische). Der Stickstoffgehalt in Mustern des erfindungsgemäßen biokompatiblen Materials schwankt zwischen 1,10 - 3,84 Masse%.

Zum besseren Verständnis der vorliegenden Erfindung werden folgende Ausführungsbeispiele des erfindungsgemäßen biokompatiblen Materials angeführt.

### Beispiel 1

Ein biokompatibles Material, bestehend aus einem Pfropfcopolymeren von α-Cyanacrylat und Polyacrylsäure mit einer Molekularmasse von 200 000 bis 600 000, das durch Umsetzung von α-Cyanacrylat mit Polyacrylsäure in Anwesenheit des Vernetzungsmittels - eines Gemisches von Polyvinylalkohol mit einer Molekularmasse von 50 000 bis 600 000 und Glycerin bei folgenden Verhältnissen der genannten Komponenten in Masse% erhalten ist:

| | |
|---|---|
| Gemisch von Ethyl-α-cyanacrylat und Ethoxyethyl-α-cyanacrylat, genommen in Verhältnis 1:1 | 19 |
| Polyacrylsäure mit einer Molekularmasse von 200 000 bis 600 000 | 35 |
| Polyvinylalkohol mit einer Molekularmasse von 50 000 bis 60 000 | 35 |
| Glycerin | 11 |

Man bereitet das genannte Material durch Vermischen einer Lösung von 0,790 g (19 Masse%) Gemisch von Ethyl-α-cyanacrylat und Ethoxyethyl-α-cyanacrylat, genommen im Verhältnis 1:1, in Chloroform und 1,41 g (35 Masse%) Polyacrylsäure mit einer Molekularmasse von 200 000 bis 600 000 in Anwesenheit von 1,41 g (35 Masse%) Polyvinylalkohol mit einer Molekularmasse von 50 000 bis 60 000 und 0,46 g (11 Masse%) Glycerin.

Nach der Begießungsmethode mit Hilfe eines Gießtrichters formt man die Folie und entfernt das Lösungsmittel bei einer Temperatur von 37 bis 39°C. Die erhaltene Folie wird danach einer Wärmebehandlung bei einer Temperatur von 100° C im Laufe von 60 Minuten ausgesetzt. Das biokompatible Material, das ein vernetztes Pfropfcopolymeres von α-Cyanacrylat mit der Polyacrylsäure darstellt, erholt man in Form einer mit Schaum bedeckten Folie mit folgenden Kennwerte:

| | |
|---|---|
| Dicke | 400 µm |
| Resorptionsfähigkeit (Anschwellung in 24 Stunden) | 1250 % |
| bezogene Dehnung | 125 % |
| Dichte | 0,36 g/cm |
| Bruchfestigkeit | 45 kp/cm² |

### Beispiel 2

Ein biokompatibles Material, bestehend aus einem Pfropfcopolymeren von Ethyl-α-cyanacrylat und Polyacrylsäure mit einer Molekularmasse von 200 000 bis 600 000, das durch Umsetzung von Ethyl-α₋cyanacrylat mit Polyacrylsäure in Anwesenheit eines Gemisches von Polyvinylalkohol mit Glycerin bei folgendem Verhältnis der Ausgangskomponenten in Masse% erhalten ist:

| | |
|---|---|
| Ethyl-α-cyanacrylat | 34 |
| Polyacrylsäure mit einer Molekularmasse von 200 000 bis 600 000 | 43 |
| Polyvinylalkohol mit einer Molekularmasse von 50 000 bis 60 000 | 21 |
| Glycerin | 2 |

Das genannte Material erhält man durch Unsetzung von 3,25 g (34 Masse%) Ethyl-α-cyanacrylat mit 4,1 g (43 Masse%) Polyacrylsäure mit der genannten Molekularmasse in Anwensenheit von 2,0 g (21 Masse%) Polyvinylalkohol und 0,19 g (2 Masse%) Glycerin. Die erhaltene Emulsion gießt man auf eine Unterlage auf, die die Bildung einer Platte mit entwickelter Oberfläche sichert. Danach wird die Unterlage zusammen mit dem Material in einem Bad untergebracht, das wäßrige Natriumchloridlösung enthält, und für 2 Stunden in dieser Lösung gelassen. Das nach dem Trocknen und der Wärmebehandlung erhaltene biokompatible Material stellt ein vernetztes Pfropfcopolymeres von Ethyl-α-cyanacrylat mit Polyacrylsäure in Form einer Platte mit entwickelter Oberfläche dar und hat folgende Kennwerte:

| | |
|---|---|
| Dicke | 30-50 mm |
| Resorptionsfähigkeit (Anschwellung in 24 Stunden) | 5000 % |
| Dichte | 0,17 g/cm |

### Beispiel 3

Ein biokompatibles Material, bestehend aus einem Pfropfcopolymeren von Butyl-α-cyanacrylat und Polyacrylsäure mit einer Molekularmasse von 200 000, bis 600 000, das durch Umsetzung von Butyl-α-cyanacrylat und Polyacrylsäure in Anwesenheit von Polyvinylalkohol bei folgendem Verhältnis der genannten Komponenten in Masse% erhalten ist:

| | |
|---|---|
| Butyl-α-cyanacrylat | 24 |
| Polyacrylsäure mit einer Molekularmasse von 200 000 bis 600 000 | 38 |
| Polyvinylalkohol mit einer Molekularmasse von 50 000 bis 60 000 | 38 |

Das genannte Material erhält man durch Umsetzung von 1,1 g (24 Masse%) Butyl-α-cyanacrylat mit 1,7 g (38 Masse%) Polyacrylsäure mit der genannten Molekularmasse in Anwensenheit von 1,7 g (38 Masse%) Polyvinylalkohol mit einer Molekularmasse von 50 000 bis 60 000. Die erhaltene Emulsion tröpfelt man beim Vermischen in ein Fällbad, in dem eine Natriumchloridlösung enthalten ist. In 3 Stunden nach der Beendigung des Tröpfelns wird das erhaltene Granulat abfiltriert und nach einer der bekannten Methoden getrocknet.

Das biokompatible Material, das ein Pfropfcopolymeres von Butyl-α-cyanacrylat mit Polyacrylsäure vernetzt durch Polyvinylalkohol, in Form von Granulat darstellt, hat folgende Kennwerte:

| | |
|---|---|
| Durchschnittsdurchmesser von Granulat | 1-2 mm |
| Resorptionsfähigkeit (Anschwellung in 24 Stunden) | 15 000 % |
| Dichte | 0,40 g/cm³ |

### Beispiel 4

Ein biokompatibles Material, bestehend aus einem Pfropfcopolymeren von Ethoxyethyl-α-cyanacrylat und Polyacrylsäure, das durch Umsetzung von Ethoxyethyl-α-cyanacrylat mit Polyacrylsäure in Anwesenheit eines Gemisches von Polyvinylalkohol mit Glycerin bei folgendem Verhältnis der Komponenten in Masse% erhalten ist:

| | |
|---|---|
| Ethyl-α-cyanacrylat | 14 |
| Polyacrylsäure mit einer Molekularmasse von 200 000 bis 600 000 | 15 |
| Polyvinylalkohol mit einer Molekularmasse von 50 000 bis 60 000 | 60 |
| Glycerin | 11 |

Das genannte Material erhält man durch Umsetzung von 1,3 g (14 Masse%) Ethoxyethyl-α-cyanacrylat mit 1,4 g (15 Masse%) Polyacrylsäure mit einer Molekularmasse von 200 000 bis 600 000 in Anwensenheit von 5,6 g (60 Masse) Polyvinylalkohol mit einer Molekularmasse von 50 000 bis 60 000 und 1,0 g (11 Masse%) Glycerin. Die Hälfte des Volumens der erhaltenen Emulsion trägt man mit Hilfe eines Gießtrichters auf eine Unterlage auf, dann legt man ein Polyethylenterephthalat-(Lavsan-)Netz mit erforderlicher Größe auf die aufgegossene Schicht und trägt die Hälfte des Emulsionsvolumens oben auf. Die Lösungsmittel entfernt man bei einer Temperatur von 37 bis 40°C, und das Material wird einer Wärmebehandlung bei einer Temperatur von 90° C im Laufe von 90 Minuten ausgesetzt.

Das biokompatible Material, das ein Pfropfcopolymeres von Ethoxyethyl-α-cyanacrylat und Polyacrylsäure, vernetzt durch Polyvinylalkohol und Glycerin, in Form einer durch Lavsannetz armierten Platte darstellt, hat folgende Kennwerte:

| | |
|---|---|
| Dicke | 1,2 mm |
| Dichte | 0,23 g/cm³ |
| Resorptionsfähigkeit (Anschwellung in 24 Stunden) | 600 % |
| Dauer der Resorption der Polymerermatrize im Muskelgewebe | 30 - 40 Tage |

### Beispiel 5

Ein biokompatibles Material, bestehend aus einem Pfropfcopolymeren von α-Cyanacrylat und Polyacrylsäure, das durch Umsetzung eines Gemisch von Ethyl-α-cyanacrylat und Ethoxyethyl-α-cyanacrylat, genommen im Verhältnis I:I, und Polyacrylsäure in Anwesenheit von Glycerin und Antiseptikum - Chlorhexydinbiglukonat (1,6-Di(N-p-chlorophenyldiguanido)hexandigluconat) bei folgendem Verhältnis der genannten Komponenten in Masse% erhalten ist:

| | |
|---|---|
| Gemisch von Ethyl-α-cyanacrylat mit Ethoxyethyl-α-cyanacrylat | 30 |
| Polyacrylsäure mit einer Molekularmasse von 200 000 bis 600 000 | 43 |
| Glycerin | 27 |
| Chlorhexydinbiglukonat | 9 (vom Gewicht aller genommenen Komponenten) |

Das genannte Material erhält man durch Umsetzung von 3,3 g (30 Masse%) Ethyl-α-cyanacrylat und Ethoxyethyl-α-cyanacrylat, genommen im Verhältnis I:I, und 4,7 g (43 Masse%) Polyacrylsäure mit einer Molekularmasse von 200 000 bis 600 000 in Anwesenheit von 3,0 g (27 Masse%) Glycerin und Clorhexyldinbiglukonat in einer Menge von 9% (0,99 g), bezogen auf die genommenen Komponenten. Durch Auspressen durch ein Propfilgießtrichter in ein Fällbad, das eine Natriumchloridlösung enthält, wird das Material in Fäden geformt. Die Lösungsmittel werden bei einer Temperatur von 37 bis 40° C entfernt.

Das biokompatible Material, das ein Pfropfcopolymeres von α-Cyanacrylat und Polyacrylsäure darstellt und das Antiseptikum-Chlorhexydinbiglukonat-enthält, hat folgende Kennwerte:

| | |
|---|---|
| Resorptionsfähigkeit (Anschwellung in 24 Stunden) Antimikrobenaktivität gegen Stämme von Staphylococcus, Colibakterium und anderen grampositiven Mikroorganismen | 700% |

### Beispiel 6

Ein biokompatibles Material, bestehend aus einem Pfropfcopolymeren von α-Cyanakrylat und Polyacrylsäure mit einer Molekularmasse von 200 000 bis 600 000, das durch Umsetzung eines Gemischs Ethyl-α-cyanacrylat und Ethoxyethyl-α-cyanacrylat, genommen im Verhältnis I:I, und Polyacrylsäure in Anwesenheit von Polyvinylalkohol und Gentamylin als Antseptikum bei folgendem Verhältnis der Ausgangskomponenten in Masse% erhalten ist.

| | |
|---|---|
| Gemisch Ethyl-α-cyanacrylat und Ethoxyethyl-α-cyanacrylat | 26 |
| Polyacrylsäure mit einer Molekularmasse von 200 000 bis 600 000 | 37 |
| Polyvinylalkohol mit einer Molekularmasse von 50 000 bis 60 000 | 37 |
| Gentamycin | 23 (vom Gewicht aller genommenen Komponenten) |

Das genannte Material erhält man durch Umsetzung von 1,8 g (26 Masse%) Ethyl-α-cyanacrylat und Ethoxyethyl-α-cyancrylat, genommen im Verhältnis I:I, 2,6 g (37 Masse%) Polyacrylsäure mit einer Molekularmasse von 200 000 bis 600 000 in Anwensenheit von 2,6 g (37 Masse%) Polyvinylalkohol mit einer Molekularmasse von 50 000 bis 60 000 und Gentamycin in einer Menge von 1,7 g (24 Masse%, bezogen auf Komponenten).

Nach der Begießungsmethode mit Hilfe eines Trichters formt man die Folie, entfernt die Lösungsmittel bei einer Temperatur von 37 bis 40° C, und setzt man das Material einer Wärmebehandlung bei einer Temperatur von 100° C im Laufe von 60 Minuten aus.

Das biokompatible Material, das ein Propfcopolymeres von α-Cyanacrylat und Polyacrylsäure darstellt und Gentamylin als Antiseptikum enthält, hat folgende Kennwerte:

| | |
|---|---|
| Resorptionsfähigkeit (Anschwellung in 24 Stunden) Antimikrobenaktivität gegen viele grampositive und gramnegative Mikroorganismen (u.a. Colibakterium, Staphylococcus und andere) | 150% |

### Industrielle Anwendbarkeit

Das erfindungsgemäße biokompatible Material zur Behandlung von Gewebe- und Organdefekten findet Anwendung in der Medizin zur Bedeckung der Bauchspeicheldrüse in Norm, bei der Pankreatitis, zur Schließung einer resektierten Oberfläche von parenchymatösen Organen, zur Substition ausgedehnter Defektstellen der oberen Bauchwand, zur Plastik des Schließapparats des Mastdarms sowie zur Behandlung von eitrigen Haut- und Operationswunden.

## Patentansprüche

1. Biokompatibles Material zur Behandlung von Gewebe- und Organdefekten auf der Grundlage eines resorptionsfähigen Polymeren, dadurch **gekennzeichnet,** daß es aus einem Pfropfcopolymeren von α-Cyanacrylat mit Polyacrylsäure mit einer Molekularmasse von 200.000 bis 600.000 besteht, das durch Umsetzung von α-Cyanacrylat mit Polyacrylsäure in Anwesenheit eines Vernetzungsmittels bei folgendem Verhältnis der Komponenten in Masse-% erhalten ist:
| | |
|---|---|
| α-Cyanacrylat | 14 bis 34 |
| Polyacrylsäure mit einer Molekularmasse von 200.000 bis 600.000 | 15 bis 43 |
| Vernetzungsmittel | 71 bis 23. |

2. Biokompatibles Material nach Anspruch 1, dadurch **gekennzeichnet,** daß es als α-Cyanacrylatglieder Ethyl-α-cyanacrylat, Ethoxyethyl-α-cyanacrylat, Butyl-α-cyanacrylat oder ihre Gemische enthält.

3. Biokompatibles Material nach einem der Ansprüche 1 oder 2, dadurch **gekennzeichnet,** daß die makromolekulare Raummatrix aus Polyvinylalkoholblöcken mit einer Molekularmasse von 50.000 bis 60.000, Glyceringliedern oder Gemischen dieser Glieder besteht.

4. Biokompatibles Material nach Anspruch 1 bis 3, dadurch **gekennzeichnet,** daß es zusätzlich ein Antiseptikum in einer Menge von 9 bis 50 Masse-% enthält.

## Claims

1. Biocompatible material for the treatment of defects in tissues and organs based on a resorbable polymer, characterized in that it consists of a grafting copolymer of α-cyanoacrylate with polyacrylic acid with a molecular mass of from 200,000 to 600,000 which is obtained by reacting α-cyanoacrylate with polyacrylic acid in the presence of a cross-linking agent with the following ratio of components in percentage by mass:
| | |
|---|---|
| α-cyanoacrylate | 14 to 34 |
| polyacrylic acid with a molecular mass of 200,000 to 600,000 | 15 to 43 |
| cross-linking agent | 71 to 23. |

2. Biocompatible material according to Claim 1, characterized in that it contains ethyl-α-cyanoacrylate, ethoxyethyl-α-cyanoacrylate, butyl-α-cyanoacrylate or mixtures thereof as α-cyanoacrylate components.

3. Biocompatible material according to one of Claims 1 or 2, characterized in that the macromolecular spatial matrix comprises polyvinylalcohol blocks with a molecular mass of from 50,000 to 60,000, glycerine components or mixtures of the said components.

4. Biocompatible material according to Claims 1 to 3, characterized in that it additionally contains an antiseptic in a quantity of from 9 to 50% by mass.

## Revendications

1. Matériau biocompatible pour traiter des défauts des tissus et des organes, à base d'un polymère résorbable, caractérisé en ce qu'il est constitué d'un copolymère greffé d'α-cyanacrylate sur un poly(acide acrylique) ayant une masse moléculaire de 200000 à 600000, qu'on obtient par réaction d'α₋cyanacrylate avec le poly(acide acrylique) en présence d'un agent de réticulation, les composants étant présents dans les proportions suivantes, en % en masse:
| | |
|---|---|
| α-cyanacrylate | 14 à 34 |
| Poly(acide acrylique) ayant une masse moléculaire de 200000 à 600000 | 15 à 43 |
| Agent de réticulation | 71 à 23. |

2. Matériau biocompatible selon la revendication 1, caractérisé en ce qu'il contient, comme motif α-cyanacrylate, de l'α-cyanacrylate d'éthyle, de l'α-cyanacrylate d'éthoxyéthyle, de l'α-cyanacrylate de butyle ou des mélanges de ceux-ci.

3. Matériau biocompatible selon l'une des revendications 1 ou 2, caractérisé en ce que la matrice spatiale macromoléculaire est constituée de séquences poly(alcool vinylique) ayant une masse moléculaire de 50000 à 60000, de motifs glycérine, ou de mélanges de ces motifs.

4. Matériau biocompatible selon les revendications 1 à 3, caractérisé en ce qu'il contient, en plus, un antiseptique en une quantité d e 9 à 5 0 % en masse.
